# EUROPÄISCHE PATENTSCHRIFT

(11) **EP 1 694 259 B1**
(45) Veröffentlichungstag und Bekanntmachung des Hinweises auf die Patenterteilung: **08.02.2017**
(21) Anmeldenummer: 04803923.4
(22) Anmeldetag: 15.12.2004
(51) Int. Cl.: A61F 9/008

(54) **LASERVORRICHTUNG ZUR BEARBEITUNG VON TRANSPARENTEM GEWEBE DES MENSCHLICHEN AUGES MITTELS LASERSTRAHLUNG**
LASER DEVICE FOR TREATING TRANSPARENT HUMAN OCULAR MATERIAL USING LASER RADIATION
DISPOSITIF À LASER DE TRAITEMENT DE TISSU OCULAIRE HUMAIN TRANSPERANT PAR FAISCEAUX LASER

(30) Priorität: 16.12.2003 DE 10358927
(43) Veröffentlichungstag der Anmeldung: 30.08.2006
(73) Patentinhaber: Carl Zeiss Meditec AG, 07745 Jena (DE)
(72) Erfinder: BISCHOFF, Mark, 07749 Jena (DE); MÜHLHOFF, Dirk, 07751 Kunitz (DE); GERLACH, Mario, 16548 Glienicke-Nordbahn (DE)
(74) Vertreter: Patentanwälte Geyer, Fehners & Partner mbB
(86) Internationale Anmeldenummer: PCT/EP2004/014309
(87) Internationale Veröffentlichungsnummer: WO 2005/058216

(56) Entgegenhaltungen:
- US-A- 4 702 245
- US-A- 5 892 569
- US-A1- 2002 021 730
- US-A1- 2003 156 615

## Beschreibung

Die Erfindung bezieht sich auf eine Laservorrichtung zur Bearbeitung von transparentem Gewebe des menschlichen Auges, mit einer gepulste Laserstrahlung bereitstellenden Laserstrahlquelle und einer variablen Ablenkeinrichtung, die die Laserstrahlung an verschiedenen, wählbaren Stellen ins Material zur Erzeugung optischer Durchbrüche einbringt.

Diese Laservorrichtung sowie das zugrunde liegende Verfahren zur Materialverarbeitung eignet sich besonders, um gekrümmte Schnittflächen innerhalb eines transparenten Materials auszubilden. Gekrümmte Schnittflächen innerhalb eines transparenten Materials werden beispielsweise bei laserchirurgischen Verfahren und dort insbesondere bei augenchirurgischen Eingriffen erzeugt. Dabei wird Behandlungs-Laserstrahlung innerhalb des Gewebes, d.h. unterhalb der Gewebeoberfläche derart fokussiert, daß optische Durchbrüche im Gewebe entstehen.

Im Gewebe laufen dann zeitlich hintereinander mehrere Prozesse ab, die durch die Laserstrahlung initiiert werden. Überschreitet die Leistungsdichte der Strahlung einen Schwellwert, kommt es zu einem optischen Durchbruch, der im Material eine Plasmablase erzeugt. Diese Plasmablase wächst nach Entstehen des optischen Durchbruches durch sich ausdehnende Gase. Anschließend wird das in der Plasmablase erzeugte Gas vom umliegenden Material aufgenommen und die Blase verschwindet wieder. Dieser Vorgang dauert allerdings sehr viel länger, als die Entstehung der Blase selbst. Wird ein Plasma an einer Materialgrenzfläche erzeugt, die durchaus auch innerhalb einer Materialstruktur liegen kann, so erfolgt ein Materialabtrag von der Grenzfläche. Man spricht dann von Photoablation. Bei einer Plasmablase, die vorher verbundene Materialschichten trennt, ist üblicherweise von Photodisruption die Rede. Der Einfachheit halber werden all solche Prozesse hier unter dem Begriff optischer Durchbruch zusammengefaßt, d.h. dieser Begriff schließt nicht nur den eigentlichen optischen Durchbruch sondern auch die daraus resultierenden Wirkungen im Material ein.

Für eine hohe Genauigkeit eines laserchirurgischen Verfahrens ist es unumgänglich, eine hohe Lokalisierung der Wirkung der Laserstrahlen zu gewährleisten und Kollateralschäden in benachbartem Gewebe möglichst zu vermeiden. Es ist deshalb im Stand der Technik üblich, die Laserstrahlung gepulst anzuwenden, so daß der zur Auslösung eines optischen Durchbruchs nötige Schwellwert für die Leistungsdichte nur in den einzelnen Pulsen überschritten wird. Die US 5.984.916 zeigt diesbezüglich deutlich, daß der räumliche Bereich des optischen Durchbruchs (in diesem Fall der erzeugten Wechselwirkung) stark von der Pulsdauer abhängt. Eine hohe Fokussierung des Laserstrahls in Kombination mit sehr kurzen Pulsen erlaubt es damit, den optischen Durchbruch sehr punktgenau in einem Material einzusetzen.

Der Einsatz von gepulster Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, daß die Brechungseigenschaften von Hornhaut und Linse keine optimale Fokussierung auf der Netzhaut bewirken.

Die erwähnte US 5.984.916 sowie die US 6.110.166 beschreiben Verfahren zur Schnitterzeugung mittels geeigneter Erzeugung optischer Durchbrüche, so daß im Endeffekt die Brechungseigenschaften der Hornhaut gezielt beeinflußt werden. Eine Vielzahl von optischen Durchbrüchen wird so aneinandergesetzt, daß innerhalb der Hornhaut des Auges ein linsenförmiges Teilvolumen isoliert wird. Das vom übrigen Hornhautgewebe getrennte linsenförmige Teilvolumen wird dann über einen seitlich öffnenden Schnitt aus der Hornhaut herausgenommen. Die Gestalt des Teilvolumens ist so gewählt, daß nach Entnahme die Form und damit die Brechungseigenschaften der Hornhaut so geändert sind, daß die erwünschte Fehlsichtigkeitskorrektur bewirkt ist. Die dabei geforderten Schnittflächen sind gekrümmt, was eine dreidimensionale Verstellung des Fokus nötig macht. Es wird deshalb eine zweidimensionale Ablenkung der Laserstrahlung mit gleichzeitiger Fokusverstellung in einer dritten Raumrichtung kombiniert. Dies wird hier unter dem Begriff "Ablenkung" subsumiert.

Beim Aufbau eines Schnittes durch Aneinanderreihung optischer Durchbrüche im Material verläuft die Erzeugung eines optischen Durchbruches um ein Vielfaches schneller, als es dauert, bis ein davon erzeugtes Plasma wieder im Gewebe absorbiert wird. Aus der Veröffentlichung A. Heistenkamp et al., Der Ophthalmologe, 2001, 98:623-628, ist es bekannt, daß nach Erzeugen eines optischen Durchbruches in der Augenhornhaut am Fokuspunkt, an dem der optische Durchbruch erzeugt wurde, eine Plasmablase wächst, die nach einigen ns eine maximale Größe erreicht und anschließend wieder nahezu vollständig kollabiert. Es bleiben dann nur kleine Restblasen übrig. Die Veröffentlichung führt aus, daß ein Zusammenschließen anwachsender Plasmablasen die Schnittqualität mindert. Es wird deshalb dort ein gattungsgemäßes Verfahren vorgeschlagen, bei dem einzelne Plasmablasen nicht direkt nebeneinander erzeugt werden. Stattdessen wird in einer spiralförmigen Bahn zwischen aufeinanderfolgend erzeugten optischen Durchbrüchen eine Lücke gelassen, die in einem zweiten Durchlauf durch die Spirale mit optischen Durchbrüchen und daraus resultierenden Plasmablasen gefüllt wird. Damit soll ein Zusammenschluß benachbarter Plasmablasen verhindert und die Schnittqualität verbessert werden.

Es ist aber generell erforderlich, den Abstand zweier aufeinanderfolgender Plasmablasen auf der Bahnkurve möglichst genau zu steuern. Im Prinzip kann dies bei konstanter Folgefrequenz der Laserpulse durch Anpassung der Bahngeschwindigkeit, also der Ablenkgeschwindigkeit, erfolgen. Im Fall der Spirale würde dies bedeuten, daß der Laserstrahl eine innere Spiralbahn wesentlich schneller (d.h. mit einer höheren Winkelfrequenz) durchläuft, als eine äußere Bahn. Dies ist eine geeignete Methode, solange die maximale Ablenkfrequenz des jeweils verwendeten Scansystems eine ausreichende Bahngeschwindigkeit zuläßt. Für die Ablenkfrequenz fₛ der für die laterale Ablenkung des Laserstrahls verwendeten Scanner gilt die einfache Beziehung fₛ=(f_{L}*s)/(2*π**r). Dabei ist f_{L} die Folgefrequenz der Pulse im gepulsten Laserstrahl und s der entlang der Bahnkurve gemessene geometrische Abstand zweier nacheinander zu erzeugender Plasmablasen auf der zumindest abschnittsweise näherungsweise kreisförmigen Bahnkurve mit dem Radius r. Nimmt man für eine Abschätzung die maximale Ablenkfrequenz üblicher Galvanometer-Scanner an, die nichtresonant bis Frequenzen von ca. 300 Hz dem Ansteuersignal mit guter Genauigkeit folgen können, so ergibt sich für s=10 µm und r=20 µm eine maximale Pulsfrequenz von etwa 4 kHz. Mit Einschränkungen hinsichtlich der Ablenkwinkel, könnten evtl. auch noch höhere Pulsfrequenzen sinnvoll verwendet werden. Allerdings wachsen dann die Positionsfehler, was einem solchen Vorgehen praktische Grenzen setzt. Diese Überlegungen zeigen, daß es mit den derzeit üblichen Scannersystemen nötig ist, zur Darstellung gewünschter Spiralbahnen die Pulsfrequenz der Laserstrahlung auf max. 10 kHz zu beschränken.

Als alternativer Ansatz wäre es theoretisch denkbar, die Pulsfrequenz der Laserstrahlung variabel zu gestalten; jedoch gibt es für ein solches Vorgehen bei der Verwendung von Lasersystemen mit passiv modensynchronisierten Oszillatoren gewisse Einschränkungen. Die heute üblichen fs-Lasersysteme liefern in medizinischer Anwendung deshalb nur Laserstrahlung mit einer festen Pulsfrequenz. Dies führt zu technischen Lösungen, die feste Pulsfrequenzen der Laserstrahlung im Bereich einiger kHz haben. Die Prozeßgeschwindigkeit bei der Schnittflächenerzeugung ist damit auf die Bereiche der Bahn abgestimmt, die die größten Anforderungen an die Ablenkung stellen.

Aus der US 2003/0156615 A1 ist ein System zum Lochbohren mittels Laserstrahlung bekannt, bei dem gepulste Leserstrahlung verwendet wird. Im Lasersystem ist außerhalb des Resonators ein Modulator vorgesehen, der einstellbar optische Verluste bewirkt, so daß die Amplitude eines Pulszuges der gepulsten Laserstrahlung verändert werden kann, um Löcher mit möglichst senkrechten Wänden su bohren.

Die bereits genannte US 5984916 sieht als Sichesheitseinrichtung eine Blende vor, die es erlaubt, den Laserstrahl abzuschalten. Dieser Sicherheitsverschluß kann mit der Intensitätssteuerung des Lasers zu einem System zusammengefaßt werden.

Bei des Augenchirurgie ist eine möglichst schnelle Schnittflächenerzeugung ist nicht nur aus Komfort- oder Zeitersparniswünschen anzustreben; vor dem Hintergrund, daß bei ophthalmologischen Operationen unvermeidlicherweise Bewegungen des Auges auftreten, fördert eine schnelle Schnittflächenerzeugung auch die optische Qualität des erzielten Resultats bzw. senkt die Anforderungen an eventuelle Nachführungen von Augenbewegungen.

Der Erfindung liegt deshalb die Aufgabe zugrunde, eine Vorrichtung der eingangs genannten Art so auszugestalten, daß für die Erzeugung einer Schnittfläche eine möglichst geringe Zeit erforderlich ist.

Die Aufgabe wird erfindungsgemäß gelöst mit einer Laservorrichtung gemäß Anspruch 1. Sie weist eine Pulsselektionseinrichtung auf, welche selektierte Laserpulse der gepulsten Laserstrahlung so hinsichtlich mindestens eines optischen Parameters verändert, daß mit den veränderten Laserpulsen keine optischen Durchbrüche erzeugbar sind.

Die Pulsfrequenz der zur Bearbeitung prinzipiell geeigneten Laserstrahlung, die von der letzten Verstärkerstufe des Lasersystems abgegeben wird, ist also konstant und wird nachträglich mittels einer geeigneten Vorrichtung physikalisch durch Beeinflussung der Laserpulse so verändert, daß nur eine Teilmenge der erzeugten Laserpulse noch im Gewebe optische Durchbrüche bewirkt. Die Laserstrahlquelle ist also im Gegensatz zum Stand der Technik hinsichtlich der Wiederholfrequenz, mit der die Laserpulse abgegeben werden, nicht mehr auf Bereiche der Bahnkurve mit den höchsten Anforderungen an die Ablenkung (z.B. größtem Abstand aufeinanderfolgender optischer Durchbrüche) optimiert, sondern kann nun sehr viel höher gewählt werde. Beispielsweise ist es möglich die Laserstrahlquelle hinsichtlich der Pulsfrequenz auf den Bereich mit den niedrigsten Anforderungen an die Ablenkung (z.B. geringstem örtlichen, beziehungsweise zeitlichem Abstand, aufeinanderfolgend zu erzeugender optischer Durchbrüche) abzustimmen. Durch die Selektion von Laserpulsen kann die hinsichtlich optischer Durchbrüche wirksame Wiederholfrequenz der Laserpulse, d.h. die Pulsfrequenz derjenigen Pulse, die in der Lage sind optische Durchbrüche auszulösen, stufenweise wählbar gemindert werden, so daß Begrenzungen des Ablenksystems nicht mehr wirksam sind. Diese Minderung durch Selektion von Laserpulsen behindert die Abstimmung und Auslegung der Laserstrahlquelle jedoch nicht, so daß die eingangs erwähnten Probleme hinsichtlich Laser mit veränderbarer Pulsfrequenz nicht auftreten.

Die Erfindung ermöglicht eine gegenseitige Abstimmung von Selektion der zu verändernden Laserpulse und Laserstrahlablenkung, so daß die Ablenkung vorteilhafterweise immer möglichst nahe der maximalen Ablenkgeschwindigkeit erfolgen kann. Dadurch ist eine schnelle Schnitterzeugung erreicht, ohne daß am Lasersystem Änderungen vorgenommen werden müßten. Für diese Abstimmung ist es natürlich wesentlich, daß die Selektion der zu verändernden Laserpulse wählbar ist.

Insbesondere ist eine variierbare Teilung der konstanten Pulsfrequenz der Laserstrahlung nach Verlassen des letzten Laserverstärkers mittels der erfindungsgemäßen Vorrichtung möglich. Es bewirkt letztlich dann nur jeder n-te Laserpuls, der den Laserverstärker verläßt, einen optischen Durchbruch im Gewebe; also z.B. jeder zweite, oder nur jeder dritte, usw. Die Teilung kann selbstverständlich variierend gestaltet werden.

Zu diesem Zweck wird durch die Pulsselektionseinrichtung der erfindungsgemäßen Vorrichtung eine Teilmenge der Laserpulse, die den letzten Laserverstärker mit hoher Pulsfrequenz verlassen, selektiert und geeignet beeinflußt. Geeignete Beeinflussung meint, daß zumindest ein physikalischer Parameter jedes selektierten Laserpulses so verändert wird, daß dieser keinen optischen Durchbruch mehr erzeugen kann; die übrigen (nicht-selektierten) Laserpulse verursachen dagegen weiterhin optische Durchbrüche im Fokuspunkt. Die selektierten Laserpulse sind also unter dem Aspekt der Wirkung im Material "unschädlich".

Als physikalischer Parameter, der erfindungsgemäß beeinflußt werden kann, kommt insbesondere die Phase, die Amplitude, die Polarisation, die Strahlrichtung (Pointing Vektor) oder die Feldverteilung über den Strahlquerschnitt (Strahlprofil) in Frage. Insbesondere können diese Parameter auch im Frequenzraum (in spektraler Darstellung) manipuliert werden, da dies bei der Veränderung ultrakurzer Pulse einfacher möglich ist. Entscheidend ist, daß die Beeinflussung der selektierten Laserpulse dazu führt, daß im Material ein Schwellwert für die Leistungsdichte, der zur Erzeugung eines optischen Durchbruchs überschritten werden muß, nicht mehr überschritten wird. Dies wird unmittelbar erreicht oder mittelbar durch nachfolgende Wechselwirkung der beeinflußten Pulse mit einem der Laserstrahlquelle nachgeordneten optischen System bzw. bestimmten Komponenten desselben.

Die gepulste Laserstrahlung wird mit einer bestimmten Pulsfrequenz erzeugt und danach hinsichtlich der selektierten Laserpulse noch verändert, wenn sie das Lasersystem (Oszillator und/oder Verstärker) bereits verlassen hat. Damit sind nachteilige Auswirkungen auf Qualität, Leistungsstabilität usw. der gepulsten Laserstrahlung vermieden und eine aufwendige Regelung des Laserverstärkers entfällt.

Das erfindungsgemäße Vorgehen nutzt zudem vorzugsweise die Schwellwertabhängigkeit der nicht-linearen Wechselwirkung zwischen Bearbeitungsstrahlung und Material, indem keine Ausblendung der selektierten und damit nicht zur Bearbeitung genutzten Laserpulse erfolgt, sondern schon eine Veränderung der selektierten Laserpulse derart genügt, daß im Material keine Bearbeitungseffekte mehr erzielt werden.

Die Beeinflussung bzw. Veränderung der selektierten Laserpulse kann unter Ausnutzung verschiedenster physikalischer Prinzipien erfolgen. Ihnen ist allen gemein, daß die optischen Kenngrößen der selektierten Laserpulse so verändert werden, daß sie ins zu bearbeitende Material gelangen und dort keinen optischen Durchbruch mehr erzeugen können. Zur Veränderung kömen beispielsweise das Prinzip der akusto-optischen Modulation, der polarisationsabhängigen Reflektion oder einer faseroptische Umschaltungen eingesetzt werden.

Die erfindungsgemäße Laservorrichtung zur Materialbearbeitung bzw. das erfindungsgemäße Verfahren zur Materialbearbeitung mittels Laserstrahlen erreicht eine Schnittflächenerzeugung, die die verfügbare Ablenkgeschwindigkeit besser ausnutzt, als dies im Stand der Technik der Fall war. Eine nahezu maximale Ausnutzung erreicht man, wenn die Ablenkgeschwindigkeit und die Pulsselektion synchron zueinander erfolgen, beispielsweise unter Eingriff einer entsprechenden Steuereinrichtung. Man kann dann die Selektion erhöhen, d.h. mehr Pulse selektieren, die keine optischen Durchbrüche erzeugen können, wenn die Ablenkung in der Nähe einer maximalen Ablenkgeschwindigkeit gerät. Durch Erhöhung der Selektion kommen innerhalb einer gegebenen Zeiteinheit weniger Pulse an der Ablenkeinrichtung an, die in der Lage sind einen optischen Durchbruch zu erzeugen. Es kann somit mit geringerer Ablenkgeschwindigkeit gearbeitet werden. Die synchrone Ansteuerung von Ablenkung und Selektion berücksichtigt dies.

Die Erfindung wird nachfolgend unter Bezugnahme auf die Zeichnungen beispielshalber noch näher erläutert. In den Zeichnungen zeigt:
- Figur 1: eine perspektivische Darstellung eines Patienten während einer Behandlung mit einem laserchirurgischen Instrument,
- Figur 2: die Fokussierung eines Strahlenbündels auf das Auge des Patienten beim Instrument der Figur 1,
- Figur 3: eine schematische Darstellung zur Erläuterung einer während der laserchirurgischen Behandlung mit dem Instrument der Figur 1 erzeugten Schnittfläche,
- Figur 4: eine Ablenkvorrichtung des laserchirurgischen Instruments der Figur 1,
- Figur 5: ein Blockdiagramm des Instrumentes der Figur 1,
- Figur 6: eine Prinzipskizze einer Ausführungsvariante eines Laserpulsmodulators des Instrumentes der Figur 1 und,
- Figur 7: Zeitreihen von Laserpulsen und elektrischen Steuersignalen für den Laserpulsmodulator der Figur 6.

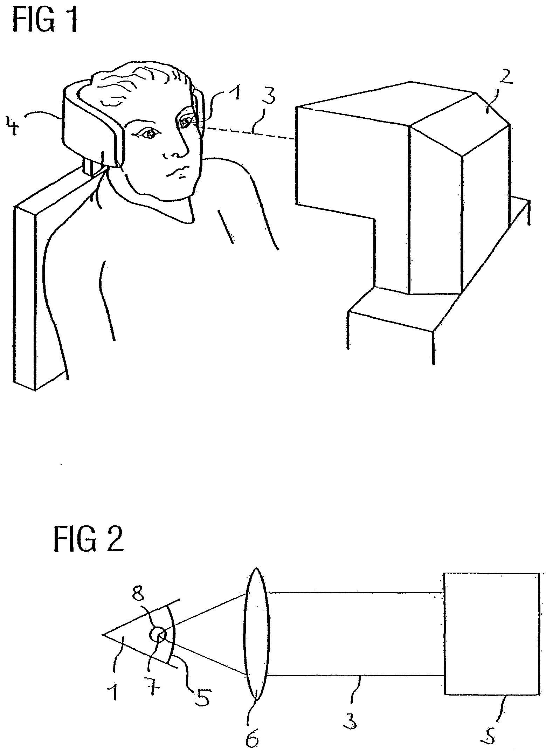

In Figur 1 ist ein laserchirurgisches Instrument zur Behandlung eines Auges 1 eines Patienten gezeigt, wobei das laserchirurgische Instrument 2 zur Ausführung einer refraktiven Korrektur dient. Das Instrument 2 gibt dazu einen Behandlungs-Laserstrahl 3 auf das Auge des Patienten 1 ab, dessen Kopf in einem Kopfhalter 4 fixiert ist. Das laserchirurgische Instrument 2 ist in der Lage, einen gepulsten Laserstrahl 3 zu erzeugen, so daß das in US 6.110.166 beschriebene Verfahren ausgeführt werden kann. Der Laserstrahl 3 besteht aus fs-Laserpulsen mit einer Pulsfrequenz zwischen 10 und 500 kHz. Die Baugruppen des Instrumentes 2 werden im Ausführungsbeispiel von einer integrierten Steuereinheit gesteuert.

Das laserchirurgische Instrument 2 weist, wie in Figur 2 schematisch dargestellt ist, eine Strahlquelle S auf, deren Strahlung in die Hornhaut 5 des Auges 1 fokussiert wird. Mittels des laserchirurgischen Instrumentes 2 wird eine Fehlsichtigkeit des Auges 1 des Patienten dadurch behoben, daß aus der Hornhaut 5 Material so entfernt wird, daß sich die Brechungseigenschaften der Hornhaut um ein gewünschtes Maß ändern. Das Material wird dabei dem Stroma der Hornhaut entnommen, das unterhalb von Epithel und Bowmanscher Membran und oberhalb von Decemetscher Membran und Endothel liegt.

Die Materialentfernung erfolgt, indem durch Fokussierung des hochenergetischen gepulsten Laserstrahls 3 mittels eines verstellbaren Teleskopes 6 in einem in der Hornhaut 5 liegenden Fokus 7 in der Hornhaut Gewebeschichten getrennt werden. Jeder Puls der gepulsten Laserstrahlung 3 erzeugt dabei einen optischen Durchbruch im Gewebe, welcher wiederum eine Plasmablase 8 initiiert. Dadurch umfaßt die Gewebeschichttrennung ein größeres Gebiet, als der Fokus 7 der Laserstrahlung 3, obwohl die Bedingungen zur Erzielung des Durchbruches nur im Fokus 7 erreicht werden. Durch geeignete Ablenkung des Laserstrahls 3 werden nun während der Behandlung viele Plasmablasen 8 erzeugt. Diese Plasmablasen bilden dann eine Schnittfläche 9, die ein Teilvolumen T des Stromas, nämlich das zu entfernende Material der Hornhaut 5 umschreiben.

Das laserchirurgische Instrument 2 wirkt durch die Laserstrahlung 3 wie ein chirurgisches Messer, das, ohne die Oberfläche der Hornhaut 5 zu verletzen, direkt Materialschichten im Inneren der Hornhaut 5 trennt. Führt man einen Schnitt 16 durch weitere Erzeugung von Plasmablasen 8 bis an die Oberfläche der Hornhaut, kann ein durch die Schnittfläche 9 isoliertes Material der Hornhaut 5 seitlich in Richtung des Pfeiles 17 herausgezogen und somit entfernt werden.

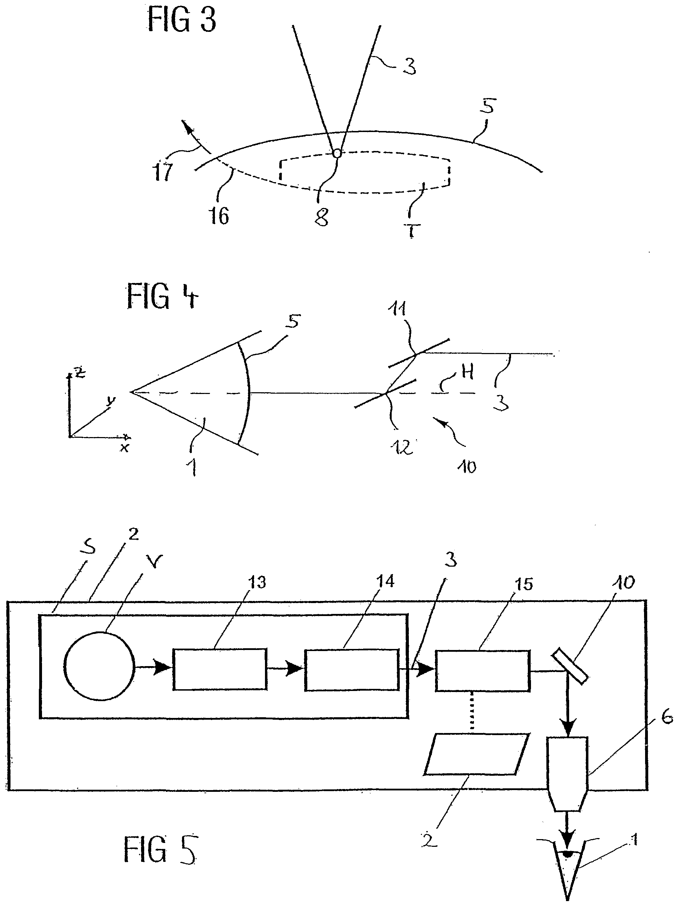

Die Erzeugung der Schnittfläche 9 mittels des laserchirurgischen Instrumentes 2 ist in Figur 3 schematisch dargestellt. Durch Aneinanderreihung der Plasmablasen 8 in Folge stetiger Verschiebung des Fokus 7 des gepulsten fokussierten Laserstrahls 3 wird die Schnittfläche 9 gebildet.

Die Fokusverschiebung erfolgt dabei zum einen in einer Ausführungsform mittels der in Figur 4 schematisch dargestellten Ablenkeinheit 10, die den auf einer Haupteinfallsachse H auf das Auge 1 einfallenden Laserstrahl 3 um zwei senkrecht zueinander liegenden Achsen ablenkt. Die Ablenkeinheit 10 verwendet dafür einen Zeilenspiegel 11 sowie einen Bildspiegel 12, was zu zwei hintereinander liegenden räumlichen Ablenkachsen führt. Der Kreuzungspunkt der Hauptstrahlachse H mit der Ablenkachse ist dann der jeweilige Ablenkpunkt. Zur Fokusverschiebung wird zum anderen das Teleskop 6 geeignet verstellt. Dadurch kann der Fokus 7 in dem in Figur 4 schematisch dargestelltem x/y/z-Koordinatensystem entlang dreier orthogonaler Achsen verstellt werden. Die Ablenkeinheit 10 verstellt den Fokus in der x/y-Ebene, wobei der Zeilenspiegel den Fokus in der x-Richtung und der Bildspiegel in der y-Richtung zu verstellen erlaubt. Das Teleskop 6 wirkt dagegen auf die z-Koordinate des Fokus 7. Somit ist insgesamt eine dreidimensionale Ablenkung des Fokus 7 erreicht.

Aufgrund der Cornea-Krümmung, die zwischen 7 und 10 mm beträgt, muß das Teilvolumen T auch entsprechend gekrümmt sein. Die Cornea-Krümmung erordert sich somit eine Bildfeldkrümmung. Diese wird durch geeignete Ansteuerung der Ablenkeinheit 10 und des Teleskopes 6 wirkt.

Die Figur 5 zeigt ein vereinfachtes Blockschaltbild des laserchirurgischen Instrumentes 2 für die refraktive Chirurgie am menschlichen Auge 1. Dargestellt sind nur die wichtigsten Details: ein als Strahlquelle S dienender fs-Laser, welcher aus einem fs-Oszillator V, sowie einer oder mehreren Verstärkerstufen 13 besteht und dem hier noch ein Kompressor bzw. Pre-Kompressor 14 nachgeordnet ist; ein Laserpulsmodulator 15, der mit der Laserstrahlung aus dem Laser S beaufschlagt wird; die Ablenkeinheit 10, hier als Scanner realisiert; ein das Teleskop 6 verwirklichendes Objektiv zur Fokussierung in das zu bearbeitende Gewebe, und die Steuereinheit 17.

Der Laser S erzeugt Laserpulse mit einer Dauer im fs Bereich. Die Laserpulse gelangen zunächst in den Laserpulsmodulator 15, der (auf noch zu beschreibende Art) eine Selektion der nicht zur Generation optischer Durchbrüche im Gewebe vorgesehenen Laserpulse vornimmt. Anschließend gelangen zumindest die nicht-selektierten Laserpulse zum Scanner 10 und durch das Objektiv 6 in das Patientenauge 1. Sie werden dort fokussiert und erzeugen im Fokus 7 optische Durchbrüche. Die selektierten Laserpulse können zwar ebenfalls zum Scanner 10 gelangen, auch weiter zum Objektiv 6 und ins Auge 1, doch unterscheiden sie sich hinsichtlich mindestens eines physikalischen Parameters derart von den übrigen Laserpulsen, daß sie im Auge 1 keinen optischen Durchbruch bewirken.

Hinsichtlich der Position des Laserpulsmodulators 15 gibt es verschiedene Möglichkeiten. Es ist mitunter vorteilhaft, ihn bereits unmittelbar nach der letzten Verstärkerstufe 13, also noch vor dem Kompressor/Pre-Kompressor 14 anzuordnen. Er kann somit auch in den Bauraum des Lasers S integriert sein, befindet sich aber immer nach der Verstärkung und dem Oszillator. Wird ein cavity-dumped Oszillator verwendet, so befindet sich der Laserpulsmodulator 15 immer innerhalb des Resonators

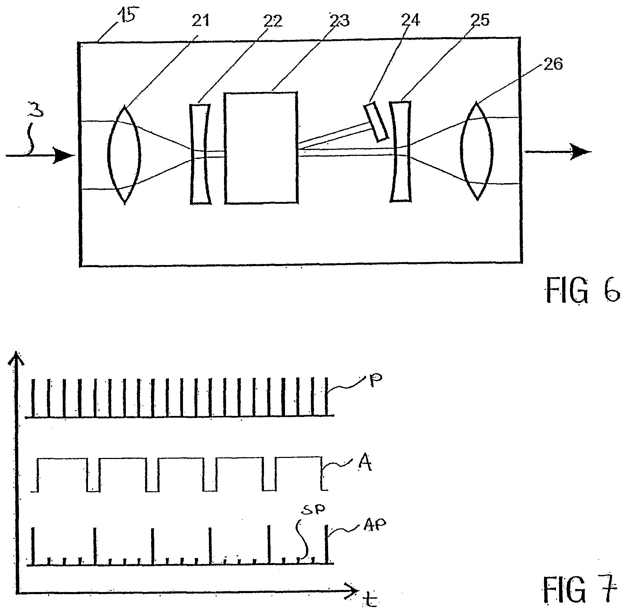

In Figur 6 ist eine Ausführungsvariante des Laserpulsmodulators 15 dargestellt. Der erzeugte Laserstrahl 3 wird zunächst mittels Linsen 21 und 22 geformt und anschließend in einen akusto-optischen Modulator 23 (AOM) geleitet. Die Linsen 21 und 22 stehen beispielhaft für zur Strahlformung geeignete optische Bauelemente (z.B. Spiegel, Linsen, DOE).

Der AOM 23 wird durch ein elektrischen Steuersignal von der Steuereinheit (hier nicht dargestellt) derart angesteuert, daß die nicht zur Erzeugung optischer Durchbrüche vorgesehenen Laserpulse selektiert werden. Der Vorgang der Selektion besteht im dargestellten Fall in einer Beugung der Laserpulse im AOM 23 und ungebeugte Transmission der übrigen Laserpulse.

Die gebeugten Laserpulse werden an einer Strahlfalle 24 absorbiert oder sind zumindest nicht mehr in der Lage, optische Durchbrüche zu bewirken. Auf die Strahlfalle 24 kann dann evtl. verzichtet werden. Die Wirkung der Überlagerung der durch die Beugung hervorgerufenen Richtungsänderung der selektierten Laserpulse mit einer Amplitudenmodulation der hier ausgeführten Variante des Laserpulsmodulators 15 besteht darin, die Pulsspitzenleistung der selektierten Laserpulse derart zu verringern, daß sie auch nach Fokussierung im Auge 1 keinen optischen Durchbruch mehr hervorrufen. Die restlichen Laserpulse bleiben im wesentlichen unverändert und erzeugen im Auge 1 optische Durchbrüche.

Eine invertierte Ausführung der erfindungsgemäßen Vorrichtung, bei der die selektierten Laserpulse einen AOM 23 ungebeugt passieren und die übrigen Laserpulse geeignet gebeugt werden, ist natürlich ebenfalls möglich.

Vorteilhaft ist an dieser Variante, daß die nicht zur Erzeugung optischer Durchbrüche vorgesehenen, selektierten Laserpulse vollständig aus dem Bearbeitungslaserstrahl entfernt werden können. Allerdings erfahren die übrigen Laserpulse beim Beugungsprozess ebenfalls einige Veränderungen, die ihre Eignung für die Materialbearbeitung verringern könnten. Diese Veränderungen stehen wesentlich mit der hohen spektralen Bandbreite ultrakurzer Laserpulse im Zusammenhang und sind oft aufwandgering kompensierbar.

Als Modulator kann anstelle des beschriebenen AOM 23 auch ein elektrooptischer Modulator (EOM), eine Pockelszelle, ein Flüssigkristallelement (LC-Element) oder ein faseroptisches Schaltelement verwendet werden, jeweils ergänzt um Bauelemente, die eine Transformation der primär veränderten optischen Eigenschaften der selektierten Laserpulse so bewirken, daß die Entstehung von optischen Durchbrüchen im Fokus verhindert ist.

Auch kann zum Zweck der Selektion beispielsweise eine zeitliche Laserpulsverlängerung (Streckung) durch Dispersion erfolgen. Dieser Effekt läßt sich beispielsweise durch eine Polarisationsdrehung der selektierten Laserpulse mittels einer geeigneten Transformation - beispielsweise durch Verwendung polarisationsabhängiger Reflektion - erreichen. Schnelle Polarisationsdrehungen kann man mit Pockelszellen herbeiführen.

Eine Wellenfrontveränderung der selektierten Laserpulse, die zu mangelhafter Fokussierung und damit zum Ausbleiben optischer Durchbrüche führt, ist natürlich ebenfalls möglich. Die selektierten Laserpulse sind dann so defokussiert, daß die Spitzenenergiedichte nicht mehr zur Initiierung optischer Durchbrüche reicht. Solche Wellenfrontveränderungen können z.B. durch Flüssigkristallelemente oder auch durch Membranspiegel, wie sie aus der adaptiven Optik bekannt sind, erzielt werden.

Die Steuereinheit 18 leistet die Ansteuerung der Laserpulsselektion. Ein geeignetes Ansteuersignal A ist in Figur 7 beispielhaft angegeben. Dargestellt ist weiter, wie die Laserpulsintensität der mit konstanter Pulsfrequenz vom letzten Laserverstärker emittierten Laserpulse P so moduliert wird, daß dadurch die gewünschte Selektion erfolgt. Die selektierten Laserpulse SP mit geringer Pulsintensität bewirken im Material keine Plasmaentstehung und die effektive Pulsfrequenz bearbeitender Laserpulse AP wird somit verringert. Im Falle der Verwendung eines AOM stellt das dargestellte Ansteuersignal A die Einhüllende des elektrischen Hochfrequenzsignals dar, mit dem der AOM betrieben wird.

Das beschriebene Konzept ist für die Anwendung bei der Lasermaterialbearbeitung, insbesondere der Mikromaterialbearbeitung mit spektral breitbandigen Laserpulsen vorteilhaft einsetzbar. Denn bei der Verwendung spektral breitbandiger Laserpulse wird eine Wirkung im Material meist dadurch erzielt, daß eine hohe Photonendichte eine nicht-lineare Wechselwirkung des Materials mit der Bearbeitungsstrahlung zur Folge hat, die wiederum eine gewünschte Veränderung im Material hervorruft. Diese nicht-lineare Wechselwirkung läßt sich besonders einfach verhindern, da sie eine starke Schwellwerteigenschaft zeigt, d.h. erst oberhalb eines Schwellwertes für die Strahlungsleistungsdichte einsetzt.

Durch Veränderung der Strahlparameter läßt sich der Bearbeitungseffekt für jeden Puls präzise auswählen. Die vorgeschlagene Vorrichtung ist auch bei der Bearbeitung nicht-organischer Materialien verwendbar, z.B. bei einer Herstellung von Wellenleiterstrukturen in transparenten Materialien. Aber auch eine Verwendung im Zusammenhang mit der Herstellung mikromechanischer Bauelemente kann vorteilhaft erfolgen.

## Patentansprüche

1. Laservorrichtung zur Bearbeitung von transparentem Gewebe des menschlichen Auges mittels nicht-linearer Wechselwirkung zwischen gepulster Laserstrahlung und dem Gewebe, mit
- einer die gepulste Laserstrahlung (3) bereitstellenden Laserstrahlquelle (S) und
- einer variablen Ablenkeinrichtung (10) und einem Objektiv (6), die die Laserstrahlung (3) an verschiedenen, wählbaren Stellen ins Gewebe (5) zur Erzeugung optischer Durchbrüche in einen Fokus (7) bündeln,
- wobei die von der Laserstrahlquelle (S) bereitgestellte gepulste Laserstrahlung (3) derart ist, daß sie im Fokus zu einer Leistungsdichte führt, die einen für das Gewebe vorbestimmten Schwellwert, oberhalb dem ein optischer Durchbruch im Gewebe entsteht, überschreitet,
- **gekennzeichnet durch**
- eine Pulsselektionseinrichtung (15), die einige Laserpulse (SP) der bereitgestellten, gepulsten Laserstrahlung (3) als selektierte Laserpulse (SP) selektiert und diese so hinsichtlich mindestens eines optischen Parameters verändert, daß für die selektierte Laserpulse (SP) die Leistungsdichte im Fokus (7) unter dem Schwellwert liegt,
- wobei die Pulsselektionseinrichtung (15) so ausgebildet ist, daß die selektierten Laserpulse (SP) zwar in den Fokus (7) gebündelt werden, jedoch dort keine optischen Durchbrüche erzeugen.

2. Laservorrichtung nach Anspruch 1, **dadurch gekennzeichnet, daß** die Pulsselektionseinrichtung (15) nicht-aufeinanderfolgende, gemäß einer Selektionsfrequenz zeitlich äquidistante Laserpulse verändert.

3. Laservorrichtung nach einem der obigen Ansprüche, **dadurch gekennzeichnet, daß** die Pulsselektionseinrichtung (15) die Laserpulse mindestens hinsichtlich einem der folgenden Parameter verändert: Phase, Amplitude, Polarisation, Ausbreitungsrichtung oder Strahlprofil.

4. Laservorrichtung nach einem der obigen Ansprüche, **dadurch gekennzeichnet, daß** die Pulsselektionseinrichtung (15) einen akusto-optischen Modulator (23), eine Pockelszelle, ein faseroptisches Schaltelement und/oder ein Chopperrad umfaßt.

5. Laservorrichtung nach einem der obigen Ansprüche, **gekennzeichnet durch** eine Steuereinrichtung (18), die die Pulsselektionseinrichtung (15) und die Ablenkeinrichtung (10) synchronisiert ansteuert.

6. Laservorrichtung nach den Ansprüchen 2 und 5, **dadurch gekennzeichnet, daß** die Steuereinrichtung (18) die Pulsselektionseinrichtung (15) und die Ablenkeinrichtung (10) zur Erzeugung der optischen Durchbrüche entlang einer vorbestimmten Bahn ansteuert und, wenn eine aktuelle Ablenkgeschwindigkeit der Ablenkeinrichtung (10) dabei nahe einer maximalen Ablenkgeschwindigkeit gerät, die Selektionsfrequenz erhöht und darauf abgestimmt die aktuelle Ablenkgeschwindigkeit herabsetzt.

## Claims

1. A laser apparatus for treatment of transparent tissue of the human eye by means of nonlinear interaction between pulsed laser radiation and the tissue, comprising
- a source of laser radiation (S) providing the pulsed laser radiation (3) and
- a variable deflecting device (10) and an objective (6) which focus said laser radiation (3) into the tissue (5) at different, selectable locations to generate optical breakthroughs in a focus (7),
- wherein the source of laser radiation (S) provides the pulsed laser radiation (3) such that it generates a power density in the focus which exceeds a threshold predetermined for the tissue, above which threshold an optical breakthrough is generated in the tissue,
**characterized by**
- a pulse picking device (15) that selects some laser pulses (SP) of the provided pulsed laser radiation (3) as selected laser pulses (SP) and modifies said selected laser pulses with regard to at least one optical parameter such that the power density in the focus (7) is below the threshold for the selected laser pulses (SP),
- wherein the pulse picking device (15) is adapted such that the selected laser pulses (SP) are focused to the focus (7), but do not cause any optical breakthroughs in it.

2. The laser apparatus according to claim 1, **characterized in that** the pulse picking device (15) modifies non-sequential laser pulses, which are equidistant in time according to a selection frequency.

3. The laser apparatus as claimed in any one of the above claims, **characterized in that** the pulse picking device (15) modifies the laser pulses at least with regard to one of the following parameters: phase, amplitude, polarization, propagation direction, or beam profile.

4. The laser apparatus as claimed in any one of the above claims, **characterized in that** the pulse picking device (15) comprises an acousto-optic modulator (23), a Pockels' cell, a fiber-optics switching element and/or a chopper wheel.

5. The laser apparatus as claimed in any one of the above claims, **characterized by** a control device (18) which synchronizes the pulse picking device (15) and the deflecting device (10).

6. The laser apparatus as claimed in claims 2 and 5, **characterized in that** the control device (18) controls the pulse picking device (15) and the deflecting device (10) so as to generate the optical breakthroughs along a predetermined path and, if an actual deflection speed of the deflecting device (10) approaches a maximum deflection speed, the control device (18) increases the selection frequency and, in accordance therewith, decreases the actual deflection speed.

## Revendications

1. Dispositif à laser destiné au traitement d'un tissu transparent de l'oeil humain au moyen d'une interaction non linéaire entre le rayonnement laser pulsé et le tissu, comprenant
- une source de rayon laser (S) qui délivre le rayonnement laser pulsé (3) et
- un appareil de déviation variable (10) et un objectif (6), qui concentrent le rayonnement laser (3) en un foyer (7) en différents endroits sélectionnables dans le tissu (5) en vue de produire des percées optiques,
- le rayonnement laser pulsé (3) délivré par la source de rayon laser (S) étant tel qu'il donne lieu, dans le foyer, à une densité de puissance qui dépasse une valeur de seuil prédéfinie pour le tissu, au-dessus de laquelle il se produit une percée optique dans le tissu,
**caractérisé par**
- un appareil de sélection d'impulsions (15) qui sélectionne certaines impulsions laser (SP) du rayonnement laser pulsé (3) délivré en tant qu'impulsions laser (SP) sélectionnées et modifie celles-ci du point de vue d'au moins un paramètre optique de telle sorte que pour les impulsions laser (SP) sélectionnées, la densité de puissance dans le foyer (7) est inférieure à la valeur de seuil,
- l'appareil de sélection d'impulsions (15) étant configuré de telle sorte que les impulsions laser (SP) sélectionnées, bien qu'elles soient concentrées en le foyer (7), n'y produisent cependant aucune percée optique.

2. Dispositif à laser selon la revendication 1, **caractérisé en ce que** l'appareil de sélection d'impulsions (15) modifie les impulsions laser non successives, équidistantes dans le temps selon une fréquence de sélection.

3. Dispositif à laser selon l'une des revendications précédentes, **caractérisé en ce que** l'appareil de sélection d'impulsions (15) modifie les impulsions laser au moins du point de vue de l'un des paramètres suivants : phase, amplitude, polarisation, sens de propagation ou profil de rayon.

4. Dispositif à laser selon l'une des revendications précédentes, **caractérisé en ce que** l'appareil de sélection d'impulsions (15) comprend un modulateur acousto-optique (23), une cellule de Pockels, un élément de commutation à fibres optiques et/ou une roue de hachage.

5. Dispositif à laser selon l'une des revendications précédentes, **caractérisé par** un appareil de commande (18) qui commande l'appareil de sélection d'impulsions (15) et l'appareil de déviation (10) de manière synchronisée.

6. Dispositif à laser selon les revendications 2 et 5, **caractérisé en ce que** l'appareil de commande (18) commande l'appareil de sélection d'impulsions (15) et l'appareil de déviation (10) en vue de produire les percées optiques le long d'une trajectoire prédéfinie et, lorsque la vitesse de déviation actuelle de l'appareil de déviation (10) s'approche ici d'une vitesse de déviation maximale, augmente la fréquence de sélection et, en adaptation avec ceci, diminue la vitesse de déviation actuelle.
